# NEUE EUROPÄISCHE PATENTSCHRIFT

(11) **EP 2 419 155 B2**
(45) Veröffentlichungstag und Bekanntmachung des Hinweises auf die Entscheidung über den Einspruch: **26.04.2017**
(45) Hinweis auf die Patenterteilung: 11.12.2013
(21) Anmeldenummer: 10719251.0
(22) Anmeldetag: 16.04.2010
(51) Int. Cl.: A61L 31/10, A61L 31/14, A61L 31/16

(54) **BESCHICHTETER STENT**
COATED STENT
STENT REVÊTU

(30) Priorität: 18.04.2009 DE 102009018013
(43) Veröffentlichungstag der Anmeldung: 22.02.2012
(73) Patentinhaber: Qualimed Innovative Medizinprodukte GmbH, 21423 Winsen (DE)
(72) Erfinder: NISSL, Martina, 21441 Garstedt (DE); GÜLCHER, Manfred, 46348 Raesfeld-Erle (DE)
(74) Vertreter: Schöneborn, Holger
(86) Internationale Anmeldenummer: PCT/EP2010/002353
(87) Internationale Veröffentlichungsnummer: WO 2010/118883

(56) Entgegenhaltungen:
- EP-A1- 1 917 971
- EP-A1- 1 980 223
- WO-A2-2004/101017
- WO-A2-2008/003298
- WO-A2-2010/118883
- DE-A1-102009 018 013
- DE-U1- 20 220 589
- DE-U1- 20 220 589
- US-A1- 2005 025 808
- US-A1- 2012 209 373
- Wikipedia-Eintrag "Carboniumion" vom 12.08.2014
- "Carboniumion", IUPAC Gold Book, Version 2.3.3, 24.02.2014
- WANG X. ET AL: 'Controlled release of sirolimus from a multilayered PLGA stent matrix' BIOMATERIALS Bd. 27, 2006, Seiten 5588 - 5595
- HOUCHIN M.L. ET AL: 'Chemical Degradation of Peptides and Proteins in PLGA: A Review of Reactions andMechanisms' JOURNAL OF PHARMACEUTICAL SCIENCES Bd. 97, Nr. 7, Juli 2008, Seiten 2395 - 2404
- AGRAWAL C.M. ET AL: 'Technique to Control pH in Vicinity of Biodegrading PLA-PGA Implants' J. BIOMED. MAT. RES. Bd. 38, Nr. 2, 1997, Seiten 105 - 114

## Beschreibung

Die Erfindung betrifft einen Stent mit einer bioresorbierbaren Polymerschicht, die mit einem die Restenose inhibierenden Wirkstoff beladen ist, wobei der Stent selbst aus einem metallischen Material besteht, das eine durch Elektropolitur geglättete und passivierte Oberfläche aufweist, in die Carbonionen implantiert sind. Der Stent ist insbesondere für den kardiovaskulären Einsatz bestimmt.

Stents werden vielfach zur Beseitigung von vaskulären Engpässen eingesetzt. Der häufigste Grund ist eine Verengung der Herzkranzgefäße durch Arteriosklerose. Eine Arteriosklerose kann eine Reihe von Ursachen haben, beispielsweise auf eine vaskuläre Verletzung zurückgehen, bei der es zu einer Hyperproliferation der vaskulären Epithelzellen kommt. In der Folge kann es zu einer mehr oder weniger starken lokalen Verengung des betroffenen Blutgefäßes kommen, bis hin zum Gefäßverschluss und zum Absterben des von dem jeweiligen Gefäßes versorgten Gewebes. Im Koronarbereich führt eine solche Verstopfung zum Herzinfarkt.

Gefäßverengungen im Bereich der Herzkrankgefäße können auf verschiedene Art und Weise behandelt werden. Zum einen besteht die Möglichkeit einer Bypass-Operation, die mit einem mehr oder weniger schweren operativen Eingriff verbunden ist. Endovaskulär können Gefäßverengungen durch Ballondilatation beseitigt werden, bei der die verengte Stelle des Gefäßes mit Hilfe eines hydraulisch aufblasbaren Ballon dilatiert wird. Die Gefäßdilatation ist zumeist auch mit der Platzierung eines Stents verbunden, bei der der Stent ("Gefäßstütze") auf den Ballon aufgekrimpt ist und mit diesem aufgeweitet und in der verengten Stelle des Gefäßes platziert wird. Der Stent stützt das Gewebe und hält im Idealfall das Gefäß offen. Diese auch als "perkutane transluminale koronare Angioplastie" (PTCA) und "Stenting" bezeichneten Behandlungsweisen geschehen auf endovaskulärem Weg und sind für den Patienten erheblich schonender, als eine operative Behandlung der Gefäßverengung.

Die mit Hilfe von aufblasbaren Ballonen gesetzten Stents werden mit einem erheblichen Druck an die Gefäßwand gepresst, zumeist mit deutlich mehr als 6 bar. Die dadurch auf die Gefäßwand ausgeübte Druckbelastung kann zu Verletzungen der Gefäßwand führen, die wiederum zu einer komplexen Kaskade an bis heute nicht vollständig bekannten Vorgängen führt. Unter anderem kommt es im Rahmen der so genannten neointimalen Hyperplasie zu der Proliferation und Migration der medialen und intimalen vaskulären glatten Muskelzellen, was letztendlich zu Anlagerung von Gewebe an dem Stent führt. Eine Restenose ist die Folge, d. h. eine erneute Gefäßverengung.

Einer solchen restenotischen Gefäßverengung versucht man auf mehreren Wegen zu begegnen, die bislang aber nur zu Teilerfolgen geführt haben. Zur Verminderung der Gefäßschäden versucht man zum einen, die Druckbelastung beim Setzen der Stents gering zu halten. Da aber ein gewisser Mindestdruck aufgewandt werden muss, um das Gefäß zu erweitern, sind diesem Ansatz Grenzen gesetzt. In bestimmten Bereichen hat man aber mit selbstexpandierenden Stents, beispielsweise aus Nitinol, Erfolge erzielt.

Ein anderer weit verbreiteter Ansatz ist die sorgfältige Glättung der Stentoberfläche durch Polierverfahren, um Verletzungen durch Grate und Unebenheiten zu vermeiden. Zu diesem Zweck werden Stents mechanisch oder elektrochemisch geglättet. Auch diesem Ansatz sind Grenzen gesetzt, da das Aufkrimpen eines Stents auf einem Ballon und die anschließende Erweiterung des Stents beim Platzieren zu Unregelmäßigkeiten an der Oberfläche führen.

Hinzu kommt, dass insbesondere im koronaren Bereich die Gefäße im Muskelgewebe eingebettet sind. Da das Muskelgewebe "arbeitet", kommt es zu einer ständigen Belastung der Gefäßwand an den gestenteten Stellen durch mechanische Reibung. Die damit einhergehende Reizung trägt zur Restenose bei.

Bei der Restenose spielt die Oberfläche, insbesondere auch die innere Oberfläche des Stents eine wichtige Rolle, als sie die Anlagerung von Zellgewebe und/oder sklerotischem Material fördern kann. Insoweit kommt es darauf an, die Stentoberfläche zum einen glatt und abweisend zu gestalten, um die Anlagerung von nicht erwünschten Materialien zu vermeiden, andererseits aber auch so zu passivieren, dass davon keine zusätzliche Reizung des verletzten Gefäßes ausgeht. Zu diesem Zweck wird beispielsweise, zusätzlich zur Elektropolitur der Stentoberfläche, eine Anreicherung der Oberfläche mit Fremdionen vorgenommen, um eine Passivierung zu erzielen. Insbesondere kann eine Carbonionenimplantierung die Metalloberfläche körperverträglicher machen und die Abgabe von toxischen Ionen vermindern, beispielsweise von Nickelionen aus nickelhaltigen medizinischen Stählen.

Um der Restenosegefahr auf anderem Wege zu begegnen, werden proliferationshemmende Medikamente eingesetzt, beispielsweise Paclitaxel oder Rapamycin. Diese Medikamente können beispielsweise durch die Verwendung damit beschichteter Ballone auf die Gefäßwand aufgebracht werden. Eine andere Möglichkeit ist die Beschichtung von Stents mit solchen proliferationshemmenden Mitteln. Hierzu wurde vorgeschlagen, den Stent mit entsprechenden Grübchen oder Kanälen zu versehen, in die das Mittel eingebracht werden kann. Häufiger eingesetzt werden aber medikamentenhaltige Beschichtungen aus biokompatiblen Polymeren (Resomere), auch solchen, die im Laufe der Zeit vom Körper resorbiert werden. Diese Beschichtungen setzen das Medikament über einen mehr oder weniger langen Zeitraum frei.

Die Praxis hat gezeigt, dass die Restenosegefahr in den ersten Tagen und Wochen nach der Gefäßdilatation am größten ist. Insoweit kommt es darauf an, ein proliferationshemmendes Medikament vor allem in dieser Zeit bereitzustellen. Viele Trägermaterialien sind aber nur beschränkt dazu geeignet, eine gleichmäßige Abgabe des Medikaments über einen definierten Zeitraum zu gewährleisten.

Ein Problem, das sich bei vielen bioresorbierbaren Polymerbeschichtungen von Stents, wie sie als Medikamententräger eingesetzt werden, ergibt, ist die Unzuverlässigkeit der Ablösung von der Stentoberfläche. Bioresorbierbare Polymere, wie sie beispielsweise unter der Bezeichnung Resomer^{®} von der Firma Boehringer Ingelheim vertrieben werden, sind polare Substanzen, die sich über Wechselwirkungen mehr oder weniger fest an die Metalloberfläche binden, insbesondere wenn diese nicht sauber poliert und passiviert ist. Auch die Tatsache, dass medizinische Stähle dazu neigen, Sauerstoff oxidativ an der Oberfläche anzulagern, führt zu einer vermehrten und festeren Bindung von polaren Polymeren an der Oberfläche. In der Folge ist die Ablösung des Polymers bzw. dessen Resorption durch den Körper nur schlecht steuerbar.

Aus der EP 1 980 223 A1 sind beschichtete Stents bekannt, bei denen auf eine DLC-Schicht eine Polymerschicht aufgebracht ist. Die Polymerschicht kann ein Homo- oder Copolymer von Milchsäure oder Glykolsäure sein.

Aus der US 2005/025808 A1 ist ein Stent beschrieben, der geeignet ist, ein therapeutisches Mittel mit polymerisationshemmender Wirkung an die Umgebung abzugeben. Der Stent kann mit Polymilchsäure oder Polyglykolsäure beschichtet sein.

Unter Berücksichtigung dieser Gesichtspunkte ist es Aufgabe der Erfindung, einen Stent bereitzustellen, der reizarm ist, dazu in der Lage ist, ein proliferationshemmendes Medikament dosiert über einen begrenzten Zeitraum aus einem resorbierbaren Polymer abzugeben und eine geringe Restenoserate aufweist. Der Stent soll eine auf Dauer angelegte hohe Körperverträglichkeit aufweisen und für den behandelnden Arzt auf üblichem Wege ohne Mehraufwand einsetzbar sein.

Diese Aufgabe wird mit einem Stent der eingangs genannten Art gelöst, bei dem die die Medikamentenlast aufnehmende Polymerbeschichtung ein Homo- oder Copolymer aus Hydroxycarbonsäuren enthält, das endständig verestert ist.

Der erfindungsgemäße Stent besteht aus einem üblichen metallischen Material, etwa einem medizinischen Stahl oder Nitinol. Der Stent selbst weist eine durch Elektropolitur geglättete Oberfläche aus, die zusätzlich passiviert ist. Die Passivierung kann dadurch erzielt werden, dass der Stent bei der Elektropolitur in einem dafür geeigneten Medium als Anode geschaltet wird.

Eine gute Elektropolitur vermag insbesondere die Rauigkeitswerte der geglätteten Oberfläche deutlich zu verbessern. Im Ergebnis erfolgt eine Annäherung der realen an die ideale Oberfläche. Mit mechanischer Glätte werden Verhältnisse der realen zur idealen Oberfläche von 100 bis 200 erreicht, die auch bei sorgfältiger Bearbeitung kaum unterschritten werden können. Eine gute Elektropolitur senkt das Verhältnis der realen zur idealen Oberfläche auf Werte, die unter 10 liegen. Ideal ist ein Verhältnis der realen zur idealen Oberfläche von 2,5 bis 5, was bedeutet, dass die für Anhaftungen auf der Stentoberfläche (nach Abtragung der Polymerbeschichtung) zur Verfügung stehende Oberfläche 2,5 bis 5 mal so groß ist wie die ideale Oberfläche. Es kommt zu weniger Anhaftungen und damit zu geringeren Restenoseraten. Zugleich entwickelt eine solchermaßen geglättete Oberfläche weniger Reizpotential auf das angrenzende Körpergewebe.

Zusätzlich zu der durch Elektropolitur geglätteten Oberfläche ist der Stent durch Ionenimplantation in seinen oberflächennahen Bereichen modifiziert. Für die Ionenimplantation haben sich Carbonionen als am besten geeignet erwiesen. Die Ionenimplantation erfolgt auf an und für sich bekannte Art und Weise. Eine Beschreibung eines Carbon-implantierten Stents mit einer geeigneten Verteilung der Carbonionen findet sich in DE 202 20 589 U1.

Bei der Carbonionenimplantation handelt es sich um die Einbringung von Kohlenstoffionen in die oberflächennahen Schichten des Stents, nicht jedoch um die Beschichtung von Stents mit Kohlenstoff. Die Ionenimplantation bewirkt die Absättigung von freien Valenzen der Metallanteile des Stents und führt zu einer Verdrängung des Sauerstoffs. Die höchsten Kohlenstoffanteile werden nicht an der Oberfläche selbst gemessen, sondern einige µm unterhalb der Oberfläche. An der Oberfläche selbst ist der Anteil an Sauerstoffatomen und insbesondere auch an Legierungszusätzen wie Nickel deutlich herabgesetzt. DE 202 20 589 A1 nutzt diesen Effekt um die Abgabe toxischer Ionen aus dem Stent an das Blut herabzusetzen und die Körperverträglichkeit der Stentoberfläche zu verbessern.

Erfindungsgemäß spielt dieser Effekt allerdings eine sekundäre Rolle. Primär kommt es darauf an, den oberflächennahen Bereich des Stents zu desaktivieren und den oberflächennahen Sauerstoffgehalt deutlich herabzusetzen. Oberflächennahe Oxide der Stentmetalle führen zur Einlagerung von Wasser und Anbindung von polaren Substanzen, insbesondere auch aus einer Polymerbeschichtung, was zur Folge hat, dass eine solche Polymerbeschichtung, insbesondere dann, wenn sie aus polaren Materialien besteht, über Wasserstoffbrückenbindungen und ggf. ionische Bindungen fest an der Oberfläche haftet. Bei bioresorbierbaren Polymerschichten, bei denen es auf eine zuverlässige Resorption und Ablösung von der Stentoberfläche ankommt, ist eine solche relativ feste Verbindung mit dem Stent für den gewünschten Zweck unerwünscht. Das Polymer haftet zu lange auf der Oberfläche des Stents, was auch dazu führt, dass die Medikamentbeladung über einen zu langen Zeitraum abgegeben wird. Eine Verminderung der Bindungsenergie zwischen Stentoberfläche und Polymer wirkt dem entgegen.

Die Carbonionenimplantation findet in der Regel nach der Elektropolitur der Stentoberfläche statt. Bei der Elektropolitur werden Unebenheiten der Oberflächen eingeebnet und dadurch auch die Gesamtoberfläche verkleinert. Wir die Elektropolitur unter oxidativen Bedingungen - in der Regel wird der zu polierende Gegenstand als Anode geschaltet - durchgeführt, führt dies gleichzeitig zur Ausfüllung einer oxidreichen Oberfläche. Die oxidreiche Oberfläche ist zum einen gut körperverträglich und zum anderen geeignet, die Abgabe von toxischen Ionen zu vermindern. Die außerordentlich glatte Oberfläche vermindert zudem die Anlagerung biologischer Materialien und insbesondere von Zellen. Andererseits kommt es zu dem oben beschriebenen Effekt der besseren Anbindung von polaren Polymerbeschichtungen.

Bioresorbierbare Polymerbeschichtungen sind in der Stenttechnologie bekannt. Vielfach werden Polyester eingesetzt. Erfindungsgemäß werden innere Polyester auf Basis von Hydroxycarbonsäuren verwandt. Diese können sowohl als Homo- als auch als Copolymere vorliegen, wobei es sich bei den Copolymeren um statistische oder um Blockcopolymere handeln kann. In jedem Fall sind die Polyester endständig zusätzlich verestert.

Polyester auf Basis von Hydroxycarbonsäuren sind grundsätzlichen bekannt und können beispielsweise durch Polymerisation oder Copolymerisation von Hydroxycarbonsäuren, deren dimeren Estern oder aus Lactonen hergestellt werden. Geeignet sind insbesondere die Hydroxycarbonsäuren mit bis zu 8 Kohlenstoffatomen, darunter Glykolsäure, Milchsäure, Hydroxybuttersäure und Hydroxyvaleriansäure. Die Polymere und Copolymere von Hydroxycarbonsäuren eignen sich aufgrund ihrer Struktur hervorragend als Medikamententräger, sind gut körperverträglich und hervorragend bioresorbierbar. Die Polyester auf Basis von Glykolsäure und Milchsäure werden vorzugsweise durch Polymerisation von Glykolid und Lactid hergestellt.

Die erfindungsgemäß zum Einsatz kommenden Polymerbeschichtungen enthalten keine freien Carboxylgruppen mehr. Vielmehr sind endständige Carbonsäuregruppen und gegebenenfalls auch seitenständige Carbonsäuregruppen im Wesentlichen alle in ihre Ester überführt. Als Ester kommen insbesondere die Ester von Alkoholen mit bis zu 6 Kohlenstoffatomen in Frage, insbesondere die Ethylester.

Die Veresterung der freien Carboxylgruppen der Polymerbeschichtung nimmt diesen die Möglichkeit zur Ausbildung von Bindungen an die Stentoberfläche, sei es durch Salzbildung oder Bildung von Wasserstoffbrückenbindungen. Zugleich wird das Reizpotential der so modifizierten Polykondensate deutlich vermindert. Insbesondere in der Frühphase einer Stentimplantation erscheint es sinnvoll, jede Form von zusätzlicher Reizung zu vermeiden.

Besonders bevorzugt sind Copolymere aus Milchsäure und Glykolsäure, etwa in einem Verhältnis von 25 bis 75 Mol-% Milchsäure und 75 bis 25 Mol-% Glykolsäure. Bevorzugt ist ein Verhältnis von 45 bis 55 Mol-% Milchsäure und 55 bis 45 Mol-% Glykolsäure, insbesondere aber ein Copolymerisat aus etwa gleichen Molanteilen. Die Milchsäure kann in ihrer D-Form oder ihrer L-Form vorliegen, vorzugsweise aber als in etwa äquimolares Gemisch aus D- und L-Milchsäure.

Die endständig veresterten Hydroxycarbonsäurepolymere können aus den unveresterten Ausgangsmaterialien, die kommerziell erhältlich sind, durch an und für sich bekannte Veresterungsverfahren hergestellt werden. Bevorzugt ist ein ethylverestertes Produkt, das aus dem Resomer^{®} RG 504 durch Veresterung erhalten wird und kommerziell verfügbar ist (Fa. Boehringer Ingelheim).

Die Polymerbeschichtung des erfindungsgemäßen Stents enthält einen die Restenose hemmenden, insbesondere proliferationshemmenden, antiangiogenen, entzündungshemmenden oder antithrombotischen Wirkstoff, wie er vielfach in der Literatur beschrieben ist. Bevorzugte Wirkstoffe sind Rapamycin und Paclitaxel. Der Wirkstoff ist auf an und für sich bekannte Weise in die Polymerbeschichtung integriert und wird durch Migrationsprozesse sowie durch den Abbau der Polymerschicht freigesetzt.

Die mit Medikament beladene Polymerschicht kann auf übliche Art und Weise auf die vorbereiteten Stents aufgebracht werden, etwa durch tauchbeschichten oder sprühbeschichten. In beiden Fällten wird der Stent durch wiederholtes Benetzen und Trocknen mit einer Lösung, die Polymer und Wirkstoff enthält mit dem gewünschten Überzung in der gewünschten Stärke überzogen.

Die Oberflächenbehandlung der erfindungsgemäßen Stents sollte so durchgeführt werden, dass nach der Carbonionenimplantation weniger als 50 % der Bindungsaktivität gegenüber der Polymerbeschichtung durch die implantierten Kohlenstoffionen gebunden sind. Vorzugsweise sollte mehr als 75 % der Bindungsaktivität gebunden sein. Die Kohlenstoffimplantation in die oberflächennahen Schichten setzt nicht nur den Anteil an toxischen (Nickel) Ionen in den grenznahen Schicht herab, sondern neutralisiert auch die Metalloxide und den eingelagerten Sauerstoff, so dass es zu einer verminderten Wassereinlagerung und einer stark verminderten Wechselwirkung zwischen den polaren Gruppen der Polymerbeschichtung und der Metalloberfläche kommt.

Zur Verteilung der Kohlenstoffionen in der Stentoberfläche wird ausdrücklich auf Fig. 2 in DE 202 29 589 U1 hingewiesen.

Insgesamt hat sich gezeigt, dass durch die Kombination einer Reihe von Behandlungsschritten der Stentoberfläche - insbesondere einer sorgfältigen Elektropolitur mit einer oxidativen Passivierung und der Carbonionenimplantation - mit einer speziellen Resomerbeschichtung, deren Carbonsäurefunktionen durch Veresterung neutralisiert wurden, ein Stent bereit gestellt werden kann, der hinsichtlich Abgabe seiner Medikamentbeladung und Abbau seiner Polymerbeschichtung den gestellten Anforderungen gerecht wird. Nach Abbau der Polymerbeschichtung bleibt eine gut körperverträgliche und die Restenose hemmende Stentoberfläche zurück, die der Einheilung in die Gefäßwand nicht entgegensteht.

## Patentansprüche

1. Stent, insbesondere für den kardiovaskulären Einsatz, mit einer bioresorbierbaren Polymerbeschichtung, die mit einem die Restenose inhibierenden Mittel beladen ist, wobei der Stent unter der Polymerbeschichtung eine durch Elektropolitur geglättete und passivierte Metalloberfläche aufweist, in die Carbonionen implantiert sind, **dadurch gekennzeichnet, dass** die Polymerbeschichtung ein Homo- oder Copolymer aus Hydroxycarbonsäuren enthält, das endständig verestert ist.

2. Stent nach Anspruch 1, **dadurch gekennzeichnet, dass** die Polymerbeschichtung aus einem Homo- oder Copolymer von Milchsäure und/oder Glykolsäure besteht.

3. Stent nach Anspruch 1 oder 2, **dadurch gekennzeichnet, dass** die Polymerbeschichtung aus 25 bis 75 Mol-% Lactideinheiten und 75 bis 25 Mol-% Glykolideinheiten besteht.

4. Stent nach Anspruch 3, **dadurch gekennzeichnet, dass** er aus 45 bis 55 Mol % Lactideinheiten und 55 bis 45 Mol-% Glycolideinheiten besteht.

5. Stent nach Anspruch 4, **dadurch gekennzeichnet, dass** die Polymerbeschichtung aus etwa 50 Mol-% D,L-Lactideinheiten und etwa 50 Mol-% Glykolideinheiten besteht.

6. Stent nach einem der vorstehenden Ansprüche, **dadurch gekennzeichnet, dass** das Polymer der Polymerbeschichtung endständig mit einem C1-C6-Alkohol verestert ist.

7. Stent nach Anspruch 6, **dadurch gekennzeichnet, dass** der Ester ein Ethylester ist.

8. Stent nach einem der vorstehenden Ansprüche, **dadurch gekennzeichnet, dass** wenigstens 50 % der Bindungsaktivität der Metalloberfläche des Stents durch Carbonionen gesättigt ist.

9. Stent nach Anspruch 8, **dadurch gekennzeichnet, dass** wenigstens 75 % der Bindungsaktivität der Metalloberfläche des Stents durch Carbonionen gesättigt ist.

10. Stent nach einem der vorstehenden Ansprüche, **dadurch gekennzeichnet, dass** die bioresorbierbare Polymerbeschichtung mit einem Wirkstoff beladen ist.

11. Stent nach Anspruch 10, **dadurch gekennzeichnet, dass** der Wirkstoff ein proliferationshemmendes Mittel ist.

12. Stent nach Anspruch 11, **dadurch gekennzeichnet, dass** der Wirkstoff Rapamycin oder Paclitaxel ist.

13. Stent nach einem der vorstehenden Ansprüche, **dadurch gekennzeichnet, dass** er aus medizinischem Stahl oder Nitinol besteht.

14. Stent nach einem der vorstehenden Ansprüche, **gekennzeichnet durch** ein Verhältnis der realen zur idealen Oberfläche von < 10 nach der Elektropolitur.

15. Stent nach Anspruch 14 mit einem Verhältnis der realen zur idealen Oberfläche von 2,5 bis 5.

## Claims

1. Stent, in particular intended for cardiovascular applications, being provided with a bioresorbable polymer coating containing a restenosis-inhibiting agent, with said stent below its polymer coating consisting of a metal surface which is smoothed by electropolishing and passivated and into which carbon ions are implanted,
**characterized in that**, the polymer coating contains a homo- or copolymer of hydroxycarboxylic acids which is terminally esterified.

2. Stent according to claim 1, **characterized in that** the polymer coating consists of a homo- or copolymer of lactic acid and/or glycolic acid.

3. Stent according to claim 1 or 2, **characterized in that** the polymer coating comprises 25 to 75 mole % of lactide units and 75 to 25 mole % of glycolide units.

4. Stent according to claim 3, **characterized in that** it comprises 45 to 55 mole % of lactide units and 55 to 45 mole % of glycolide units.

5. Stent according to claim 4, **characterized in that** the polymer coating consists of approximately 50 mole % of D,L-lactide units and approximately 50 mole % of glycolide units.

6. Stent according to any one of the above claims, **characterized in that** the polymer of the polymer coating is terminally esterified with a C1-C6 alcohol.

7. Stent according to claim 6, **characterized in that** the ester is an ethyl ester.

8. Stent according to any one of the above claims, **characterized in that** at least 50 % of the bonding activity of the stent's metal surface is saturated by carbon ions.

9. Stent according to claim 8, **characterized in that** at least 75 % of the bonding activity of the stent's metal surface is saturated by carbon ions.

10. Stent according to any one of the above claims, **characterized in that** an active agent has been incorporated into the bioresorbable polymer coating.

11. Stent according to claim 10, **characterized in that** the active agent is a proliferation-inhibiting agent.

12. Stent according to claim 11, **characterized in that** the active agent is Rapamycin or Paclitaxel.

13. Stent according to any one of the above claims, **characterized in that** it consists of medical steel or nitinol.

14. Stent according to any one of the above claims, **characterized by** a ratio of < 10 of the real in relation to the ideal surface after electropolishing.

15. Stent according to claim 14, with a ratio between the real and ideal surface ranging between 2.5 and 5.

## Revendications

1. Stent, notamment pour application cardiovasculaire, avec un revêtement en polymère biorésorbable comportant une substance inhibant la resténose, le stent présentant en-dessous du revêtement en polymère une surface métallique lissée et passivée par polissage électrolytique et dans laquelle sont implantés des ions de carbone,
**caractérisé en ce que** le revêtement en polymère comprend un homopolymère ou copolymère d'acides alpha-hydroxylées estérisé en fin de chaîne.

2. Stent selon la revendication 1, **caractérisé en ce que** le revêtement en polymère est un homopolymère ou copolymère d'acide lactique et/ou d'acide glycolique.

3. Stent selon la revendication 1 ou 2, **caractérisé en ce que** le revêtement en polymère comporte 25 à 75 mole pourcent d'unités de lactide et 75 à 25 mole pourcent d'unités de glycolide.

4. Stent selon la revendication 3, **caractérisé en ce que** le revêtement en polymère comporte 45 à 55 mole pourcent d'unités de lactide et 55 à 45 mole pourcent d'unités de glycolide.

5. Stent selon la revendication 4, **caractérisé en ce que** le revêtement en polymère comporte environ 50 mole pourcent d'unités de lactide D, L et environ 50 mole pourcent d'unités de glycolide.

6. Stent selon l'une quelconque des revendications précédentes, **caractérisé en ce que** le polymère du revêtement polymère est estérisé en fin de chaîne avec un alcool C1, C6.

7. Stent selon à revendication 6, **caractérisé en ce que** l'ester est un ester éthylique.

8. Stent selon l'une quelconque des revendications précédentes, **caractérisé en ce qu'**au moins 50 % de l'activité de liaison de la surface métallique du stent est saturée par des ions de carbone.

9. Stent selon la revendication 8, **caractérisé en ce qu'**au moins 75 % de l'activité de liaison de la surface métallique du stent est saturée par des ions de carbone.

10. Stent selon l'une quelconque des revendications précédentes, **caractérisé en ce que** le revêtement biorésorbable est chargé d'un principe actif.

11. Stent selon la revendication 10, **caractérisé en ce que** le principe actif est une substance retardant la prolifération.

12. Stent selon la revendication 11, **caractérisé en ce que** le principe actif est la rapamycine ou le paclitaxel.

13. Stent selon l'une quelconque des revendications précédentes, **caractérisé en ce qu'**il est en acier médical ou en nitinol.

14. Stent selon l'une quelconque des revendications précédentes, **caractérisé par** un rapport auprès polissage électrolytique inférieur à 10 entre la surface réelle et la surface idéale.

15. Stent selon la revendication 14, **caractérisé par** un rapport de 2,5 à 5 entre la surface réelle et la surface idéale.
